# EUROPÄISCHE PATENTANMELDUNG

(11) **EP 1 700 618 A1**
(43) Veröffentlichungstag der Anmeldung: **13.09.2006**
(21) Anmeldenummer: 06003881.7
(22) Anmeldetag: 25.02.2006
(51) Int. Cl.: A61Q 19/00, A61Q 19/04, A61Q 17/04, A61K 8/02, A61K 8/37, A61K 8/39, A61K 8/34, A61K 8/36

(54) **Langzeitstabile kosmetische Emulsionen**

(30) Priorität: 11.03.2005 DE 102005011785
(71) Anmelder: Goldschmidt GmbH, 45127 Essen (DE)
(72) Erfinder: Allef, Petra, 53121 Bonn (DE); Hameyer, Peter, 45138 Essen (DE); Meyer, Jürgen, 48143 Münster (DE); Polak, Gabriele, 58099 Hagen (DE)

(57) **Zusammenfassung**

Gegenstand der Erfindung sind langzeitstabile, niedrigviskose, feinteilige Öl-in-Wasser-Emulsionen, enthaltend eine Emulgatorkombination aus Nichtkohlenhydrat-Polyolpartialestern von linearen oder verzweigten, gesättigten oder ungesättigten Fettsäuren mit 6 bis 22 C-Atomen (Emulgatorkomponente A) und Emulgatoren auf Kohlenhydratbasis (Emulgatorkomponente B), ein oder mehrere Öle sowie Konservierungsmittel, die Herstellung der Emulsionen aus Konzentraten, die entsprechenden Konzentrate sowie die Verwendung der erfindungsgemäßen Emulsionen zur Herstellung von kosmetischen, dermatologischen oder pharmazeutischen Zubereitungen, insbesondere zur Herstellung von Tränkemulsionen für Feuchttücher (Wet Wipes).

## Beschreibung

Gegenstand der Erfindung sind langzeitstabile, niedrigviskose, feinteilige Öl-in-Wasser-Emulsionen, deren Herstellung aus Konzentraten, die entsprechenden Konzentrate sowie die Verwendung der erfindungsgemäßen Emulsionen zur Herstellung von kosmetischen, dermatologischen oder pharmazeutischen Zubereitungen, insbesondere zur Herstellung von Tränkemulsionen für Feuchttücher (Wet Wipes).

Emulsionen stellen einen wichtigen Produkttyp im Bereich kosmetischer, dermatologischer und/oder pharmazeutischer Zubereitungen dar. Kosmetische Zubereitungen werden im Wesentlichen zur Hautpflege benutzt. Hautpflege im kosmetischen Sinn ist in erster Linie, dass die natürliche Funktion der Haut als Barriere gegen Umwelteinflüsse (beispielsweise Schmutz, Chemikalien, Mikroorganismen) und gegen den Verlust von körpereigenen Stoffen (beispielsweise Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist ferner, den durch tägliches Waschen verursachten Fett- und Wasserverlust der Haut auszugleichen und die Weichheit und Glätte der Haut zu erhalten bzw. wieder herzustellen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind schützen und die Hautalterung verzögern. Die Summe dieser Hautpflegeeffekte wird in der Kosmetik allgemein unter dem Begriff "skin conditioning" zusammengefasst.

Kosmetische Zubereitungen werden auch als Desodorantien verwendet. Pharmazeutische topische Zusammensetzungen enthalten in der Regel ein oder mehrere Medikamente in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (beispielsweise Kosmetikverordnung, Lebensmittel- und Arzneimittelgesetz).

In den letzten Jahren gewannen kosmetische Feuchttücher aufgrund ihrer überaus einfachen und bequemen Verwendbarkeit zunehmende Bedeutung. Zunächst waren nahezu ausschließlich Feuchttücher für Reinigungszwecke auf dem Kosmetikmarkt vertreten, die hauptsächlich wässrige, tensidische Tränklösungen enthielten. Mehr und mehr erschienen aber zuletzt auch Pflegeprodukte auf dem Markt, die auf Tränkemulsionen basieren und somit zusätzlich eine pflegende Ölkomponente enthalten.

Die meisten dieser kosmetischen Feuchttücher für Körper- und Gesichtspflege sind dabei mit Emulsionen getränkt, die nach der PIT-Emulgiermethode (K. Shinoda, H. Kunieda, Phase properties of emulsions: PIT and HLB, Encycl. of Emulsion Technology, 337-367 (1), 1983 oder Th. Förster, F. Schambil, W. von Rybinski, J. Disp. Sci. And Technology, 13(2), 183-93 (1992)) hergestellt wurden. Die PIT-Methode macht sich die Tatsache zunutze, dass in einer Öl-in-Wasser(O/W)-Emulsion, die durch nichtionische, Polyethylenglykol enthaltende Emulgatoren stabilisiert ist, durch Temperaturerhöhung eine Phaseninversion zu einer Wasser-in-Öl(W/O)-Emulsion induziert werden kann (Phaseninversion; PIT: Phaseninversionstemperatur). Da in diesem Phaseninversionsbereich die Grenzflächenspannung Wasser-Öl extrem gering ist, können so nach Abkühlung extrem feinteilige Öl-in-Wasser Emulsionen erhalten werden. Dazu ist allerdings notwendig, dass für jedes zu emulgierende System die einzelnen Komponenten der Emulsionen genau aufeinander abgestimmt werden. Das heißt, Emulgatormischungen und Emulgatorkonzentration müssen für unterschiedliche Ölphasen "maßgeschneidert werden".

Die so hergestellten feinteiligen und niedrigviskosen Emulsionen besitzen eine ausgezeichnete Langzeitstabilität und eignen sich somit gut als Tränklösungen für Feuchttücher. Derartige Systeme sind beispielsweise in EP-B-1 268 740 oder in WO-A-00/04230 beschrieben.

Ein kommerziell angebotenes Emulsionskonzentrat, das mit Hilfe der PIT-Technologie hergestellt wird, ist Emulgade® CM der Firma Cognis (Düsseldorf), das auf den ethoxylierten Emulgatoren Ceteareth-20 und Ceteareth-12 basiert und Cetylisononanoat als Ölkomponente enthält. Dieses Konzentrat kann mit Wasser auf die erwünschte Einsatzkonzentration verdünnt werden. Die verdünnte Emulsion kann dann als Tränklösung für Feuchttücher eingesetzt werden.

Nachteilig bei diesen Tränklösungen für Feuchttücher auf Basis von PIT-Emulsionen ist, dass sie ethoxylierte Emulgatoren enthalten. Im Zuge von möglichst natürlichen Kosmetikformulierungen ist ein wichtiges Ziel der Kosmetikforschung, auf Polyethylenglykol ("PEG") enthaltende Emulgatoren verzichten zu können. Daher besteht eine verstärkte Suche nach PEG-freien Alternativlösungen.

Weiterhin ist bekannt, dass ethoxylierte Emulgatoren ein eher wässriges Hautgefühl vermitteln, was durch den Einsatz von beispielsweise Polyglycerinestern sensorisch verbessert werden muss.

So beschreibt WO-A-02/056841 PEG-freie Tränkemulsionen für kosmetische Feuchttücher auf Basis von Polyolpoly-12-hydroxystearaten und Alkylglykosiden. Eine kommerziell erhältliche Kombination dieser Emulgatoren für den beschriebenen Anwendungszweck bildet das Produkt Eumulgin® VL 75 (Cognis). Der Einsatz dieser Emulgatormischungen führt zu einem verbesserten Weichgriff der damit getränkten Papierprodukte und führt auch ansonsten zu verbesserten sensorischen Eigenschaften bei Verwendung der damit hergestellten Feuchttücher.

Sehr wichtig bei der Herstellung von Feuchttüchern ist eine ausreichende Konservierung der Tränklösungen, um Verkeimungen vorzubeugen. Dabei muss die Konservierung ausreichend sein, dass sowohl die Tränklösungen selbst, sowie schließlich auch die getränkten Feuchttücher gegenüber Verkeimungen geschützt sind.

Als bevorzugte Konservierungsmischungen werden dabei in Tränklösungen typischerweise Mischungen von Alkylparabenestern und Phenoxyethanol eingesetzt, wie sie etwa unter den Handelsnamen Euxyl® K 300 (Schülke & Mayr) oder Phenonip® (Clariant) kommerziell erhältlich sind.

Die beschriebenen hohen Anforderungen bzgl. einer sicheren Konservierung von Tränklösung und Feuchttüchern machen es dabei notwendig, dass in der Regel relativ große Mengen dieser Alkylparabenester/Phenoxyethanol-Mischungen in den fertigen Tränklösungen eingesetzt werden müssen (0,5 bis 1,0 Gew.-%). Idealerweise sollte bei der Herstellung von Emulsionskonzentraten bereits die gesamte Menge an Konservierungsmittel eingearbeitet sein. Dies erlaubt, dass die gewünschte Einsatzkonzentration der Tränklösung anschließend einfach durch Verdünnung mit Wasser eingestellt werden kann.

Berücksichtigt man, dass der Ölphasengehalt von Tränklösungen typischerweise im Bereich von 3 bis 10 Gew.-% liegt, so erkennt man, dass in 50 bis 80 %igen Konzentraten etwa 5 bis 15 Gew.-% Euxyl® K 300 oder Phenonip® enthalten sein müssen, um eine ausreichende Konservierung zu gewährleisten.

Es ist bekannt, dass der Einsatz dieser Alkylparabenester/Phenoxyethanol-Mischungen emulsionsbelastenden Einfluss hat, da diese Verbindungen sehr grenzflächenaktiv sind und mit Emulgatormolekülen um einen Platz an der Grenzfläche Öl-Wasser konkurrieren.

Verstärkt wird dieser emulsionsbelastende Effekt im Fall von Tränkemulsionen für Feuchttücher durch die notwendigen hohen Mengen dieser Konservierungsmittel und die geringen Viskositäten der Tränklösungen. Dies macht die Herstellung stabiler feinteiliger Emulsionen, die ausreichende Mengen Konservierungsmittel enthalten, sehr schwierig.

So zeigte sich etwa, dass es mit der in der WO-A-02/056841 beschriebenen Emulgatorkombination (z.B. Eumulgin® VL 75) unter Einarbeitung der notwendigen Mengen der genannten Konservierungsmittel nicht möglich war, Tränklösungen mit ausreichender Stabilität zu erhalten (siehe Vergleichsbeispiele).

Somit bestand hoher Bedarf, PEG-freie Emulgatorsysteme zur Verfügung zu stellen, die die Formulierung von Tränklösungen zur Herstellung kosmetischer Feuchtpflegetücher mit einer ausreichenden Menge Konservierungsmittel erlauben. Weiterhin sollten die Emulgatorsysteme die Formulierung einer Vielzahl unterschiedlicher Öle erlauben.

Überraschenderweise wurde nun gefunden, dass durch Kombination von Emulgatoren auf Polyglycerinbasis mit Emulgatoren auf Kohlenhydratbasis stabile, feinteilige und dünnflüssige PEG-freie Emulsionen erhalten werden können, die auch mit den notwendigen Mengen an Konservierungsstoffen, insbesondere Alkylparabenester/Phenoxyethanol ausgezeichnet langzeit- und lagerstabil sind. Diese Emulsionen eignen sich darüber hinaus hervorragend zur Herstellung von Tränklösungen für Feuchtpflegetücher. Sie zeichnen sich insbesondere durch ihre einfache Herstellung und ihren feinen Dispersionsgrad aus.

Die mit Hilfe dieser Tränklösungen hergestellten Feuchttücher besitzen einen angenehmen Weichgriff und zeichnen sich darüber hinaus durch überaus angenehme sensorische Eigenschaften aus.

Ein Gegenstand der Erfindung ist eine Öl-in-Wasser-Emulsion, enthaltend eine Emulgatorkombination aus Nichtkohlenhydrat-Polyolpartialestern von linearen oder verzweigten, gesättigten oder ungesättigten Fettsäuren mit 6 bis 22 C-Atomen (Emulgatorkomponente A) und Emulgatoren auf Kohlenhydratbasis (Emulgatorkomponente B), ein oder mehrere Öle, mindestens 10 Gew.-% Konservierungsmittel bezogen auf die Gesamtmenge an Emulgatorkomponenten A und B.

Mit der erfindungsgemäßen Öl-in-Wasser-Emulsion ist erstmals eine PEG-freie, niedrigviskose Emulsion verfügbar, die trotz hohen Anteils an Konservierungsstoffen langzeitstabil ist und sich so für den Einsatz als beispielsweise Tränkemulsion für Feuchttücher eignet.

Ein weiterer Gegenstand der Erfindung ist daher eine PEG-freie (d.h. keine ethoxylierten Bestandteile enthaltende) Öl-in-Wasser-Emulsion, enthaltend eine Emulgatorkombination aus Nichtkohlenhydrat-Polyolpartialestern von linearen oder verzweigten Fettsäuren mit 6 bis 22 C-Atomen (Emulgatorkomponente A) und Emulgatoren auf Kohlenhydratbasis (Emulgatorkomponente B), ein oder mehrere Öle sowie Konservierungsmittel.

Kohlenhydrate sind definitionsgemäß Polyhydroxyaldehyde (Aldosen) u. Polyhydroxyketone (Ketosen) sowie höhermolekulare Verbindungen, die sich durch Hydrolyse in solche Verbindungen überführen lassen. Sie haben meist die Bruttoformel CnH2nOn oder Cₙ(H₂O)ₙ. Die monomeren Polyhydroxyaldehyde oder Polyhydroxyketone werden als Monosaccharide, ihre Dimeren bis Decameren Oligosaccharide (Disaccharide, Trisaccharide usw.) und die makromolekularen Kohlenhydrate als Polysaccharide bezeichnet. Die Mono- und Oligosaccharide werden als "Zucker" zusammengefasst und den Polysacchariden gegenübergestellt.

### Die Monosaccharide zeigen folgende gemeinsame Reaktion:

Kupfer-, Silber- und Wismut-Salze werden in Lösung reduziert, Blausäure wird addiert, mit Phenylhydrazin entstehen Osazone.

Nichtkohlehydrate im Sinne der vorliegenden Erfindung sind daher Polyole, die nicht der Definition der Kohlenhydrate entsprechen und nicht ihre Reaktion zeigen. Einige der erfindungsgemäß mitverwendbaren Polyole können aber durch Reduktion aus ihnen hergestellt werden, wie beispielsweise die Zuckeralkohole oder deren Anhydride wie beispielsweise Sorbitane (Monoanhydrosorbite).

Die erfindungsgemäße Öl-in-Wasser-Emulsion enthält bevorzugt 20 bis 75 Gew.-% Konservierungsmittel, bezogen auf die Gesamtmenge an Emulgatorkomponenten A und B.

Die erfindungsgemäßen Öl-in-Wasser-Emulsionen können zusätzlich gegebenenfalls Co-Emulgatoren und übliche Hilfs- und Zusatzstoffe enthalten.

Bei der Emulgatorkomponente A handelt es sich besonders bevorzugt ausschließlich oder teilweise um Polyglycerinpartialester, die beispielsweise durch Umsetzung von Polyglycerinen mit linearen oder verzweigten Fettsäuren mit 6 bis 22 C-Atomen erhältlich sind. Das eingesetzte Polyglyceringemisch weist vorteilhaft einen mittleren Kondensationsgrad von 2 bis 10, vorzugsweise von 2 bis 8 und besonders bevorzugt von 3 bis 6 auf.

Der Veresterungsgrad des eingesetzten Polyglyceringemischs liegt dabei vorteilhaft zwischen 5 und 70 %, vorzugsweise zwischen 10 und 30 %, bezogen auf die ursprünglichen Hydroxylgruppen des Polyols.

Falls gewünscht kann die Emulgatorkomponente A teilweise oder vollständig beispielsweise durch Sorbitanmono- und/oder -diester von ungesättigten und gesättigten Fettsäuren mit 6 bis 22 C-Atomen gebildet werden. Bevorzugt wird dabei Sorbitanmonooleat (etwa das Handelsprodukt TEGO® SMO V) eingesetzt.

Bevorzugt kann dabei Emulgatorkomponente A neben einem oder mehreren Polyglycerinpartialestern bis zu 50 Gew.-% Sorbitanester enthalten, bezogen auf die Gesamtmenge an Emulgatorkomponente A. Abhängig vom Ölsystem ist teilweise sogar ein ausschließlicher Einsatz von Sorbitanester als Emulgatorkomponente A bevorzugt. Dies kann jedoch auch zu weniger feinteiligen Tränkemulsionen führen.

Bei der Emulgatorkomponente B handelt es sich um eine oder mehrere verschiedene Emulgatoren auf Kohlenhydratbasis, die bevorzugt aus den folgenden Gruppen ausgewählt sind:
i) Ester aus Mono- und/oder Polysacchariden und einer oder mehreren linearen oder verzweigten Fettsäuren mit 6 bis 22 C-Atomen.
ii) Glykoside aus Mono- oder Polysacchariden und linearen oder verzweigten Fettalkoholen mit 6 bis 22 C-Atomen. Beispiele für solche Produkte sind Alkylpolyglykoside, wie 1-Octyl-, 1-Decyl-, 1-Lauryl-, 1-Myristyl-, 1-Cetyl- und 1-Stearyl-Alkylpolyglykosid. Üblicherweise können die Fettalkohole auch aus natürlichen Fetten und Ölen gewonnen werden, wodurch eine Verteilung verschiedener Alkylketten nebeneinander vorliegen kann. Neben den oder anstelle von Alkylresten können auch Alkenylreste eingesetzt werden. Die erfindungsgemäß eingesetzten Alkylpolyglykoside enthalten im Schnitt bevorzugt 1 bis 5 Zuckereinheiten, besonders bevorzugt 1,1 bis 2 Zuckereinheiten.
iii) Glykoside, die zusätzlich mit linearen oder verzweigten Fettalkoholen mit 1 bis 22 C-Atomen, verethert worden sind. Üblicherweise können die Fettalkohole auch aus natürlichen Fetten und Ölen gewonnen werden, wodurch eine Verteilung verschiedener Kettenlängen nebeneinander vorliegen kann.
iv) Glykosidester aus Mono- oder Polysacchariden und linearen oder verzweigten Fettalkoholen mit 1 bis 22 C-Atomen und einer oder mehreren linearen oder verzweigten Fettsäuren mit 1 bis 22 C-Atomen. Dabei können sowohl Fettsäuren, als auch Fettalkohole aus natürlichen Fetten und Ölen gewonnen sein und somit eine Kettenlängenverteilung aufweisen.

Als Zuckerbaustein können beliebige Mono- oder Oligosaccharide eingesetzt werden. Üblicherweise werden Zucker mit 5 bzw. 6 Kohlenstoffatomen sowie die entsprechenden Oligosaccharide eingesetzt. Solche Zucker können beispielsweise Glucose, Fructose, Galactose, Arabinose, Ribose, Xylose, Lyxose, Allose, Altrose, Mannose, Gulose, Idose, Talose und Sucrose sein.

Als Fettsäurekomponente werden bevorzugt Caprylsäure, Heptansäure, Caprinsäure, Nonansäure, Isononansäure, Laurinsäure, Tridecansäure, Myristinsäure, Palmitinsäure, Stearinsäure, Isostearinsäure, Arachinsäure und Behensäure sowie Mischungen hieraus verwendet. Natürlich vorkommende Mischungen sind beispielsweise die Kokosfettsäuren, die als Hauptbestandteil Laurinsäure, daneben noch gesättigte C₁₄- bis C₁₈-Fettsäuren und gegebenenfalls gesättigte C₈- bis C₁₀-Fettsäuren und ungesättigte Fettsäuren enthalten, sowie Talgfettsäuren, welche im Wesentlichen eine Mischung aus Palmitinsäure und Stearinsäure darstellen.

Als zusätzliche ungesättigte Fettsäurekomponenten eignen sich monoolefinisch ungesättigte Säuren, beispielsweise Hexadecensäuren, Octadecensäuren, wie Ölsäure (cis-9-Octadecensäure) oder Elaidinsäure (trans-9-Octadecensäure), Eicosensäuren und Docosensäuren, wie Erucasäure (cis-13-Docosensäure) oder Brassidinsäure (trans-13-Docosensäure), mehrfach ungesättigte Fettsäuren, beispielsweise Octadecadiensäuren und Octadecatriensäuren, wie Linolsäure und Linolensäure, sowie Mischungen hieraus.

Ebenfalls geeignet sind die bei Raumtemperatur flüssigen Fettsäuren wie Öl-, Ricinol-, Eruca- und Isostearinsäure, die 18 bis 22 Kohlenstoffatome enthalten. Ihre Erstarrungspunkte liegen aufgrund einer Verzweigung oder einer Doppelbindung in der Kohlenwasserstoffkette unter 35 °C. Verwendbar sind weiterhin Fettsäuregemische, die auch wachsartige Komponenten, wie gehärtete Ricinolsäure, enthalten können.

Als Alkoholkomponente werden bevorzugt verwendet: Methanol, Ethanol, Propanol, Isopropanol, Butanol, Pentanol, Hexanol, Heptanol, Octanol, Nonanol, Isononylalkohol, Decanol, Laurylalkohol, Myristylalkohol, Cetylalkohol, Stearylalkohol. Ebenfalls geeignet sind ungesättigte Fettalkohole wie Oleylalkohol und verzweigte Alkohole wie Guerbet-Alkohol.

Dabei werden die Emulgatorkomponenten A und B in einem Gewichtsverhältnis von 9:1 bis 1:9, bevorzugt von 8:2 bis 2:8 und besonders bevorzugt von 7:3 bis 3:7 eingesetzt, wobei annähernde Parität im Bereich von 3:2 bis 2:3 oder 5:4 bis 4:5 optimal ist.

Die Gesamtmenge der eingesetzten Emulgatorkomponenten A + B bewegt sich dabei bevorzugt bezogen auf die zu emulgierende Ölmenge in einer Spanne von 15 bis 70 Gew.-%, bevorzugt von 25 bis 55 Gew.-% und besonders bevorzugt von 30 bis 50 Gew.-%.

Als geeignete Konservierungsmittel werden besonders bevorzugt Mischungen einzelner oder mehrerer Alkylparabenester mit Phenoxyethanol eingesetzt. Vorzugsweise handelt es sich bei den Alkylparabenestern um Methlyparaben, Ethylparaben, Propylparaben und/oder Butylparaben, beziehungsweise deren Alkoholate, insbesondere Natriumalkoholate. Anstelle von Phenoxyethanol können auch andere Alkohole verwendet werden, wie beispielsweise Benzylalkohol oder Ethanol.

Darüber hinaus können auch andere übliche Konservierungsmittel wie etwa Sorbin- oder Benzoesäure, Salicylsäure, 2-Bromo-2-Nitropropan-1,3-Diol, Chloracetamid, Diazolidinyl Harnstoff, DMDM Hydantoin, Iodopropynyl Butylcarbamat, Natrium Hydroxymethylglycinate oder die Kombination Chlormethyl-/Methylisothiazolin eingesetzt werden.

Bei Anteilen von mindestens 10 Gew.-% an Konservierungsmitteln ist in der Regel eine ausreichende Konservierung erreichbar. Erfindungsgemäß bevorzugt sind Konzentrationen ≥ 20 Gew.-% bis ca. 75 Gew.-%. Höhere Anteile sind möglich, aber aus den oben genannten Gründen nicht bevorzugt.

In der erfindungsgemäßen Öl-in-Wasser-Emulsion enthaltene Öle können beispielsweise kosmetische Öle sein. Als kosmetische Öle kommen insbesondere Mono- oder Diester von linearen und/oder verzweigten Mono- und/oder Dicarbonsäuren mit 2 bis 44 C-Atomen mit linearen und/oder verzweigten gesättigten oder ungesättigten Alkoholen mit 1 bis 22 C-Atomen in Betracht. Ebenso sind im erfindungsgemäßen Sinne die Veresterungsprodukte aliphatischer, difunktioneller Alkohole mit 2 bis 36 C-Atomen mit monofunktionellen aliphatischen Carbonsäuren mit 1 bis 22 C-Atomen geeignet. Als Ölkomponenten geeignete Monoester sind beispielsweise die Methylester und Isopropylester von Fettsäuren mit 12 bis 22 C-Atomen wie beispielsweise Methyllaurat, Methylstearat, Methyloleat, Methylerucat, Isopropylpalmitat, Isopropylmyristat, Isopropylstearat, Isopropyloleat. Andere geeignete Monoester sind beispielsweise n-Butylstearat, n-Hexyllaurat, n-Decyloleat, Isooctylstearat, Isononylpalmitat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat sowie Ester, die aus technischen aliphatischen Alkoholschnitten und technischen, aliphatischen Carbonsäuregemischen erhältlich sind, beispielsweise Ester aus ungesättigten Fettalkoholen mit 12 bis 22 C-Atomen und gesättigten und ungesättigten Fettsäuren mit 12 bis 22 C-Atomen wie sie aus tierischen und pflanzlichen Fetten zugänglich sind. Geeignet sind aber auch natürlich vorkommende Monoester- bzw. Wachsester-Gemische wie sie beispielsweise im Jojobaöl oder im Spermöl vorliegen.

Geeignete Dicarbonsäureester sind beispielsweise Di-n-butyl-adipat, Di-n-butyl-sebacat, Di-(2-ethylhexyl)-adipat, Di-(2-hexyldecyl)-succinat, Diisotridecylacelat. Geeignete Diolester sind beispielsweise Ethylenglycoldioleat, Ethylenglycol-di-isotridecanoat, Propylenglycol-di-(2-ethylhexanoat), Butandiol-di-isostearat und Neopentylglycol-dicaprylat. Weiterhin können Ester der Benzoesäure wie C₁₂₋₁₅₋Alkylbenzoat oder auch Carbonate, vorzugsweise Dialkylcarbonate wie Dicaprylylcarbonat, Diethylhexylcarbonat und Diisononylcarbonat verwendet werden.

Als Ölkomponente können auch Fettsäuretriglyceride verwendet werden, wobei unter diesen die natürlich vorkommenden Öle und Fette bevorzugt sind. So kommen beispielsweise natürliche, pflanzliche Öle, beispielsweise Olivenöl, Sonnenblumenöl, Sojaöl, Erdnussöl, Rapsöl, Mandelöl, Palmöl aber auch die flüssigen Anteile des Kokosöls oder des Palmkernöls sowie tierische Öle wie beispielsweise Klauenöl, die flüssigen Anteile des Rindertalgs oder auch synthetische Triglyceride von Capryl-Caprinsäure-Gemischen, Triglyceride aus technischer Ölsäure oder aus Palmitinsäure-Ölsäure-Gemischen als Ölkomponenten in Frage. Weiterhin können Kohlenwasserstoffe, insbesondere auch flüssige Paraffine und Isoparaffine eingesetzt werden. Weiterhin sind auch Fettalkohole wie Oleylalkohol oder Octyldodecanol, sowie Fettalkoholether wie Dicaprylylether einsetzbar. Geeignete Siliconöle und -wachse sind z.B. Polydimethylsiloxane, Cyclomethylsiloxane, sowie aryl- oder alkyl- oder alkoxy-substituierte Polymethylsiloxane oder Cyclomethylsiloxane, wobei diese Siliconöle nicht mehr als 50 Gew.-% der eingesetzten Ölphase ausmachen sollten.

Besonders bevorzugte Ölkomponenten sind die kosmetischen Esteröle Ethylhexylpalmitat, Ethylhexylstearat, Decylcocoat, Diethylhexylcarbonat, Dioctylcarbonat, Cetearylethylhexanoat, Cetearylisononanoat, Hexyllaurat, Isopropylisononanoat, Isopropylpalmitat, Isopropylmyristat und Isopropyllaurat.

Insgesamt können die erfindungsgemäßen Formulierungen vorteilhaft 1 bis 25 Gew.-% Ölphase (Öl + Emulgator), insbesondere 1,5 bis 10 Gew.-% Ölphase und besonders bevorzugt 2 bis 6 Gew.-% Ölphase enthalten.

Neben den beschriebenen Emulgatorkomponenten können zusätzlich weitere Emulgatoren oder Tenside in den erfindungsgemäßen Emulsionen enthalten sein. Dabei handelt es sich vorzugsweise um nichtionische, anionische, kationische oder amphotere Tenside.

Als nichtionogene Tenside kommen Verbindungen aus mindestens einer der folgenden Gruppen in Frage:
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C_{12/18}-Fettsäuremono- und -diester von Anlagerungsprodukten von 1 bis 30 Mol Ethylenoxid an Glycerin,
- Glycerinmono- und -diester und Sorbitanmono- und -diester von gesättigten und ungesättigten Fettsäuren mit 6 bis 22 Kohlenstoffatomen und deren Ethylenoxidanlagerungsprodukte,
- Ethoxylierte Alkylmono- und -oligoglycoside mit 8 bis 22 Kohlenstoffatomen im Alkylrest,
- Anlagerungsprodukte von 15 bis 200 Mol Ethylenoxid an Ricinusöl und/oder gehärtetes Ricinusöl,
- Polyol- und insbesondere Polyglycerinester, wie beispielsweise Polyglycerinpolyricinoleat, Polyglycerin-Poly-12-hydroxystearat oder Polyglycerindimerat. Ebenfalls geeignet sind Gemische von Verbindungen aus mehreren dieser Substanzklassen,
- Anlagerungsprodukte von 2 bis 15 Mol Ethylenoxid an Ricinusöl und/oder gehärtetes Ricinusöl,
- Partialester auf Basis linearer, verzweigter, ungesättigter bzw. gesättigter C_{6/22}-Fettsäuren, Ricinolsäure sowie 12-Hydroxystearinsäure und Glycerin, Polyglycerin, Pentaerythrit, Dipentaerythrit, Zuckeralkohole (beispielsweise Sorbit), Alkylglucoside (beispielsweise Methylglucosid, Butylglucosid, Laurylglucosid) sowie Polyglucoside (beispielsweise Cellulose),
- Mono-, Di- und Trialkylphosphate sowie Mono-, Di- und/oder Tri-PEG-alkylphosphate und deren Salze,
- Polysiloxan-Polyether-Copolymere (Dimethicone Copolyole), wie z.B. PEG/PPG-20/6 Dimethicone, PEG/PPG-20/20 Dimethicone, Bis-PEG/PPG-20/20 Dimethicone, PEG-12 oder PEG-14 Dimethicone, PEG/PPG-14/4 oder 14/12 oder 20/20 oder 18/18 oder 17/18 oder 15/15. Besonders geeignet sind hierbei Produkte wie Bis-PEG/PPG-14/14 Dimethicone (mit Cyclopentasiloxan: ABIL® EM 97) oder insbesondere PEG/PPG-16/16 Dimethicone (mit Caprylic/Capric Triglyceride: ABIL® Care 85),
- Polysiloxan-Polyalkyl-Polyether-Copolymere bzw. entsprechende Derivate, wie z.B. Lauryl oder Cetyl Dimethicone Copolyole, insbesondere Cetyl PEG/PPG-10/1 Dimethicone (ABIL® EM 90),
- Mischester aus Pentaerythrit, Fettsäuren, Citronensäure und Fettalkohol gemäß DE-B 11 65 574 und/oder Mischester von Fettsäuren mit 6 bis 22 Kohlenstoffatomen, Methylglucose und Polyolen, vorzugsweise Glycerin oder Polyglycerin.

Auch können anionische Tenside zusätzlich verwendet werden. Diese enthalten wasserlöslichkeits-vermittelnde, anionische Gruppen wie z.B. eine Carboxylat-, Sulfat-, Sulfonat- oder Phosphat-Gruppe und einen lipophilen Rest. Hautverträgliche anionische Tenside sind dem Fachmann in großer Zahl bekannt und im Handel erhältlich. Dabei handelt es sich insbesondere um Alkylsulfate oder Alkylphosphate in Form ihrer Alkali, Ammonium- oder Alkanolammoniumsalze, Alklyethersulfate, Alklyethercarboxylate, Acylsarkosinate sowie Sulfosuccinate und Acylglutamate in Form ihrer Alkali oder Ammoniumsalze.

Auch kationische Tenside können zugesetzt werden. Als solche sind insbesondere quartäre Ammoniumverbindungen verwendbar, etwa Alkltrimethylammoniumhalogenide wie z.B. Cetyltrimethylammoniumchlorid oder -bromid oder Behenyltrimethylammoniumchlorid, aber auch Dialkyldimethylammoniumhalogenide wie z.B. Distearyldimethylammoniumchlorid. Weiterhin können Monoalklyamidoquats wie z.B. Palmitamidopropyltrimethylammoniumchlorid oder entsprechende Dialkylamidoquats eingesetzt werden. Weiterhin können biologisch gut abbaubare quarternäre Esterverbindungen eingesetzt werden, bei denen es sich meist um quaternierte Fettsäureester auf Basis von Mono-, Di- oder Triethanolamin handelt. Weiterhin können Alkylguanidiniumsalze als kationische Emulgatoren beigesetzt sein.

Der Zusatz dieser kationischen Tenside zu den erfindungsgemäßen Zusammensetzungen kann zu einer deutlichen Verbesserung des Weichgriffs der Feuchtpflegetücher sorgen.

Weiterhin ist es möglich, amphotere Tenside wie z.B. Betaine, Amphoacetate oder Amphopropionate zu den erfindungsgemäßen Zusammensetzungen zuzugeben.

Weiterhin können die erfindungsgemäßen Öl-in-Wasser Emulsionen übliche Hilfs- und Zusatzstoffe enthalten wie Konsistenzgeber, Verdickungsmittel, Wachse, UV-Lichtschutzfilter, Antioxidantien, Hydrotrope, Deodorant- und Antitransparantwirkstoffe, Insektrepellentien, Selbstbräuner, Parfümöle, Farbstoffe und biogene Wirkstoffe.

Als Konsistenzgeber kommen in erster Linie Fettalkohole oder Hydroxyfettalkholole mit 12 bis 22 und vorzugsweise 16 bis 18 Kohlenstoffatomen und daneben Partialglyceride, Fettsäuren oder Hydroxyfettsäuren in Betracht.

Geeignete Verdickungsmittel sind beispielsweise Polysaccharide, insbesondere Xanthan-Gum, Guar und Guarderviate, Agar-Agar, Alginate und Tylosen, Cellulose und Cellulosederivate, wie z.B. Carboxymethylcellulose, Hydroxyethylcellulose, Hydroxymethlypropylcellulose, außerdem alkylmodifizierte Zuckerderivate wie z.B. Cetylhydroxyethylcellulose, ferner höhermolekulare Polyethylenglycolmono- und diester von Fettsäuren, Carbomere (vernetzte Polyacrylate), Polyacrylamide, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside.

Unter UV-Lichtschutzfiltern, die in den erfindungsgemäßen Emulsionen enthalten sein können, sind organische Substanzen zu verstehen, die in der Lage sind, ultraviolette Strahlen zu absorbieren und die aufgenommene Energie in Form längerwelliger Strahlung, beispielsweise Wärme wieder abzugeben. Verwendbare UV-B-Filter können öllöslich oder wasserlöslich sein. Als verwendbare öllösliche UV-B-Filter sind beispielsweise zu nennen:
- 3-Benzylidencampher und dessen Derivate, beispielsweise 3-(4-Methylbenzyliden)campher,
- 4-Aminobenzoesäurederivate, vorzugsweise 4-(Dimethylamino)benzoesäure-2-ethylhexylester, 4-(Dimethylamino)benzoesäure-2-ethylhexylester und 4-(Dimethylamino)benzoesäureamylester,
- Ester der Zimtsäure, vorzugsweise 4-Methoxyzimtsäure-2-ethylhexylester, 4-Methoxyzimtsäureisopentylester, 2-Cyan-3-phenyl-zimtsäure-2-ethylhexylester (Octocrylene),
- Ester der Salicylsäure, vorzugsweise Salicylsäure-2-ethylhexylester, Salicylsäure-4-isopropylbenzylester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon,
- Ester der Benzalmalonsäure, vorzugsweise 4-Methoxybenzalmalonsäuredi-2-ethylhexyester,
- Triazinderivate, wie beispielsweise 2,4,6-Trianilino-(p-carbo-2'-ethyl-1'-hexyloxy)-1,3,5-triazin und Octyltriazon,
- Propan-1,3-dione, wie beispielsweise 1-(4-tert.Butylphenyl)-3-(4'methoxyphenyl)propan-1,3-dion.

Als verwendbare wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze,
- Sulfonsäurederivate von Benzophenon, vorzugsweise 2-Hydroxy-4-methoxybenzophenon-5-sulfonsäure und ihre Salze,
- Sulfonsäurederivate des 3-Benzylidencamphers, wie beispielsweise 4-(2-Oxo-3-bornylidenmethyl)benzolsulfonsäure und 2-Methyl-5-(2-oxo-3-bornyliden)sulfonsäure und deren Salze.

Als typische, erfindungsgemäß einsetzbare UV-A-Filter kommen insbesondere Derivate des Benzoylmethans in Frage, wie beispielsweise 1-(4'tert.Butylphenyl)-3-(4'-methoxyphenyl)propan-1,3-dion oder 1-Phenyl-3-(4'-isopropylphenyl)propan-1,3-dion. Die UV-A- und UV-B-Filter können selbstverständlich auch in Mischungen eingesetzt werden. Neben den genannten löslichen Stoffen kommen für diesen Zweck auch unlösliche Pigmente, nämlich feindisperse Metalloxide bzw. Salze in Frage, wie beispielsweise Titandioxid, Zinkoxid, Eisenoxid, Aluminiumoixid, Ceroxid, Zirkoniumoxid, Silicate (Talk), Bariumsulfat und Zinkstearat. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Eine relativ neue Klasse von Lichtschutzfiltern sind micronisierte organische Pigmente, wie beispielsweise 2,2'-Methylene-bis-{6-(2H-benzotriazole-2-yl)-4-(1,1,3,3-tetramethylbutyl)-phenol) mit einer Partikelgröße von kleiner 200 nm, das beispielsweise als 50 %ige wässrige Dispersion erhältlich ist.

Neben den beiden vorgenannten Gruppen primärer Lichtschutzstoffe können auch sekundäre Lichtschutzmittel vom Typ der Antioxidantien eingesetzt werden, die die photochemische Reaktionskette unterbrechen, welche ausgelöst wird, wenn UV-Strahlung in die Haut eindringt. Typische Beispiele hierfür sind Superoxid-Dismutase, Tocopherole (Vitamin E) und Ascorbinsäure (Vitamin C). Weitere geeignete UV-Lichtschutzfilter sind der Übersicht von P. Finkel in SÖFW-Journal 122, 543 (1996) zu entnehmen.

Zur Verbesserung des Fließverhaltens und der Anwendungseigenschaften können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Typische Beispiele sind:
- Glycerin,
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton,
- Technische Oligoglyceringemische mit einem Eigenkondensationsgrad von 1,5 bis 10 wie etwa technische Diglyceringemische mit einem Diglyceringehalt von 40 bis 50 Gew.-%,
- Methylolverbindungen wie insbesondere Trimethylolethan, Trimethylolpropan, Trimethylolbutan, Pentaerythrit und Dipentaerythrit,
- Niedrigalkylgucoside, insbesondere solche mit 1 bis 4 Kohlenstoffatomen im Alkylrest wie beispielsweise Methyl- und Butylglucosid,
- Zuckeralkohole mit 5 bis 12 Kohlenstoffatomen wie beispielsweise Sorbit oder Mannit,
- Zucker mit 5 bis 12 Kohlenstoffatomen wie beispielsweise Glucose oder Saccharose,
- Aminozucker wie beispielsweise Glucamin.

Als Deodorantwirkstoffe kommen beispielsweise Geruchsüberdecker, wie die gängigen Parfümbestandteile, Geruchsabsorber, beispielsweise die in der Patentoffenlegungsschrift DE-40 09 347 beschriebenen Schichtsilikate, von diesen insbesondere Montmorillonit, Kaolinit, Illit, Beidelit, Nontronit, Saponit, Hectorit, Bentonit, Smectit, ferner beispielsweise Zinksalze der Ricinolsäure, in Frage. Keimhemmende Mittel sind ebenfalls geeignet, in die erfindungsgemäßen Öl-in-Wasser-Emulsionen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-198 55 934, DE-37 40 186, DE-39 38 140, DE-42 04 321, DE-42 29 707, DE-42 29 737, DE-42 38 081, DE-43 09 372, DE-43 24 219 und EP-666 732 beschriebenen wirksamen Agenzien. Weitere übliche Antitranspirantwirkstoffe können ebenfalls vorteilhaft in den erfindungsgemäßen Zubereitungen verwendet werden, insbesondere Adstringentien, beispielsweise basische Aluminiumchloride wie Aluminiumchlorhydrat ("ACH") und Aluminium-Zirkonium-Glycine-Salze ("ZAG").

Als Insekten-Repellentien kommen N,N-Diethyl-m-toluamid, 1,2-Pentandiol oder Insect Repellent 3535 in Frage.

Als Selbstbräuner eignen sich z.B. Dihydroxyaceton und Erythrulose.

Als Parfümöle seien genannt Gemische aus natürlichen und synthetischen Riechstoffen. Natürliche Riechstoffe sind

Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln, (Macis, Angelica, Sellerie, Kardamon, Costus, Iris, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethyl-phenylglycinat, Allylcyclohexylpropionat, Styrallylpropionat und Benzylsalicylat. Zu den Ethern zählen beispielsweise Benzylethylether, zu den Aldehyden beispielsweise die linearen Alkanale mit 8 bis 18 Kohlenstoffatomen, Citral, Citronellal, Citronellyloxyacetaldehyd, Cyclamenaldehyd, Hydroycitronellal, Lilial und Bourgeonal, zu den Ketonen beispielsweise die Jonone, α-Isomethylionon und Methyl-cedrylketon, zu den Alkoholen Anethol, Citronellol, Eugenol, Isoeugenol, Geraniol, Linalool, Phenylethylalkohol und Terpineol, zu den Kohlenwasserstoffen gehören hautpsächlich die Terpene und Balsame. Bevorzugt werden jedoch Mischungen verschiedener Riechstoffe verwendet, die gemeinsam eine ansprechende Duftnote erzeugen. Auch ätherische Öle geringer Flüchtigkeit, die meist als Aromakomponenten verwendet werden, eignen sich als Parfümöle, beispielsweise Salbeiöl,

Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romillat, Irotyl und Floramat allein oder in Mischungen eingesetzt.

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S. 81 bis 106 zusammengestellt sind. Diese Farbstoffe werden üblicherweise in Konzentrationen von 0,001 bis 0,1 Gew.-%, bezogen auf die gesamte Mischung, eingesetzt.

Unter biogenen Wirkstoffen sind beispielsweise Tocopherol, Tocopherolacetat, Tocopherolpalmitat, Ascorbinsäure, Desoxyribonucleinsäure, Retinol, Bisabolol, Allantoin, Phytantriol, Panthenol, AHA-Säuren, Aminosäuren, Hyaluronsäure, Creatin (und Creatinderivate), Guanidin (und Guanidinderivate), Ceramide, Phytosphingosin (und Phytosphingosinderivate), Sphingosin (und Sphingosinderivate), Pseudoceramide, essentielle Öle, Peptide, Proteinhydrolysate, Pflanzenextrakte und Vitaminkomplexe zu verstehen.

Ein weiterer Gegenstand der Erfindung ist ein Verfahren zur Herstellung feinteiliger, niedrigviskoser Öl-in-Wasser Emulsionen mit der zuvor beschriebenen Zusammensetzung, wobei man zunächst bei erhöhten Temperaturen ein Emulsionskonzentrat herstellt und anschließend mit Wasser verdünnt.

So wurde überraschenderweise gefunden, dass über die Zwischenstufe eines mikroemulsionsähnlichen Konzentrats, das bei erhöhten Temperaturen unter minimaler Rührleistung hergestellt werden kann und durch anschließende Verdünnung dieses Konzentrats, sehr feinteilige Emulsionen erhalten werden können, die sich durch eine ausgezeichnete Lagerstabilität auszeichnen, obwohl größere Mengen Akylparabenester/Phenoxyethanol als Konservierungsmittel enthalten sind.

Dabei zeigte sich eine klare Tendenz, dass nur solche Emulsionen feindispers und somit langzeitstabil hergestellt werden konnten, die als Emulsionskonzentrat bei erhöhten Temperaturen eine transparent-klare oder nahezu transparente mikroemulsionsähnliche Phase auszubilden vermochten.

Emulsionen, die bei 40 bis 60 °C ein milchig-trübes Aussehen aufweisen, sind in der Verdünnung auf Anwendungskonzentration aufgrund eines zu groben Dispersionsgrades nicht mehr langzeitstabil bei Lagerung und liegen außerhalb der Erfindung.

Die als Vorstufe für feindisperse, niedrigviskose Emulsionen benötigten Emulsionskonzentrate können im Labormaßstab mit Hilfe einfachen, manuellen Rührens auf einer Heizplatte hergestellt werden. Die Emulsionskonzentrate enthalten dabei 10 bis 70 Gew.-% bevorzugt 20 bis 50 Gew.-% Wasser, sie sind somit 30 bis 90 %ig, bevorzugt 50 bis 80%ig.

Typischerweise werden die erfindungsgemäßen Emulsionskonzentrate bei Temperaturen zwischen 40 und 85 °C hergestellt, indem die Wasserphase portionsweise in die Ölphase eingerührt wird, die im Wesentlichen aus Emulgatorgemisch, Öl und Konservierungsmittel besteht. Parfumöle werden typischerweise bei etwa 40 °C zu diesem Emulsionskonzentrat zugegeben. Sie werden in der Regel klar solubilisiert. Je nach Kombination von Emulgatorgemisch, Öl und Konservierungsmittelmenge und gegebenenfalls Hilfs- und Zusatzstoffen werden bei erfindungsgemäßen Systemen transparente oder nahezu transparente mikroemulsionsartige Phasen in einem Teil des oben beschriebenen Temperaturbereiches, insbesondere im Bereich von 50 bis 65 °C beobachtet.

Diese Emulsionskonzentrate sind bei Abkühlung nicht lagerstabil (Phasenseparation) und müssen somit spätestens an der unteren Temperaturgrenze des mikroemulsionsähnlichen Bereichs verdünnt werden. Dabei ist es vorteilhaft, die Emulsionskonzentrate derart zu verdünnen, dass sie in vorgelegtes Wasser eingerührt werden, dessen Temperatur maximal 15 °C gegenüber der unteren Temperaturgrenze des mikroemulsionsähnlichen Bereichs erniedrigt sein sollte. Bevorzugt werden die beschriebenen Emulsionskonzentrate auf die Art und Weise verdünnt, dass sie in eine vorgelegte Wasserphase von 20 bis 50 °C eingerührt werden.

Dabei zeigte sich eine klare Tendenz, dass nur solche Emulsionen feindispers und somit langzeitstabil hergestellt werden konnten, die als Emulsionskonzentrat bei erhöhten Temperaturen eine transparent-klare oder nahezu transparente mikroemulsionsähnliche Phase auszubilden vermochten.

Emulsionen die bei 40 bis 60 °C ein milchig-trübes Aussehen aufweisen, sind in der Verdünnung auf Anwendungskonzentration aufgrund eines zu groben Dispersionsgrades nicht mehr langzeitstabil bei Lagerung und liegen außerhalb der Erfindung.

Langzeitstabil im Sinne der Erfindung bedeutet, dass die Emulsionen ohne erkennbare bzw. nennenswerte Phasentrennung 3 Monate bei Raumtemperatur (25 °C) und ca. 1 Monat bei 40 °C gelagert werden können.

Ein weiterer Gegenstand der Erfindung ist daher ein im Bereich von 50 bis 65 °C im Wesentlichen transparentes Konzentrat, enthaltend 30 bis 90 Gew.-%, besonders bevorzugt 50 bis 80 Gew.-% einer Ölphase, bestehend im Wesentlichen aus Ölkomponente, Emulgatorkomponenten und Konservierungsmittel. und ad 100 Gew.-% einer Wasserphase, wobei das Konzentrat im Wesentlichen PEG-frei ist.

Ein weiterer Gegenstand der Erfindung ist die Verwendung der erfindungsgemäßen Emulsionen zur Herstellung von kosmetischen, dermatologischen oder pharmazeutischen Zubereitungen. Insbesondere steht dabei der Einsatz als Tränklösungen zur Herstellung von Feuchttüchern, ganz besonders von kosmetischen Feuchttüchern zur Pflege und Reinigung der Haut im Vordergrund.

Ein weiterer Gegenstand der Erfindung ist die Verwendung der Emulsionen zur Herstellung von kosmetischen Reinigungs- und Pflegezubereitungen für Haut- und Hautanhangsgebilde.

Ein weiterer Gegenstand der Erfindung ist die Verwendung der Emulsionen zur Herstellung von Sonnenschutzformulierungen und von selbstbräunenden Zubereitungen, die vorzugsweise Dihydroxyaceton oder Erythrulose enthalten. Insbesondere steht dabei der Einsatz als Tränklösungen zur Herstellung von kosmetischen Feuchttüchern für Sonnenschutz oder für selbstbräunende Anwendungen im Vordergrund.

Ein weiterer Gegenstand der Erfindung ist die Verwendung der Emulsionen zur Herstellung von Reinigungs- und Pflegemitteln für harte Oberflächen. Insbesondere steht dabei der Einsatz als Tränklösungen zur Herstellung von Feuchttüchern zur Pflege und Reinigung harter Oberflächen im Vordergrund.

### Ausführungsbeispiele:

Die folgenden Beispielemulsionen sollen den Gegenstand der Erfindung näher erläutern, ohne ihn auf diese Beispiele einzuschränken.
Die Konzentrationsangaben in allen Beispielen sind als Gew.-% angegeben.
In allen Anwendungsbeispielen wurden die Parfümöle bei 40 bis 45 °C zu den Emulsionskonzentraten zugegeben, bevor diese mit Wasser verdünnt wurden.

### Emulsionen 1 bis 5:

Die Emulsionen 1 bis 5 zeigen insbesondere, dass die in der WO-A-02/056841 beschriebene Emulgatorkombination aus Polyglycerin-2 Dipolyhydroxystearate und Lauryl Glucoside nicht in der Lage ist, mit der für eine ausreichende Konservierung von Feuchttüchern benötigten Menge von
Alkylparabenestern und Phenoxyethanol (Phenonip®) (Phenoxyethanol, Methylparaben, Ethylparaben, Butylparaben, Propylparaben, Isobutylparaben) stabile Tränkemulsionen zu erzeugen. Im Vergleich dazu wird gezeigt,

dass eine erfindungsgemäße Emulgatorkombination diese Aufgabe erfolgreich bewältigen kann.

Zum besseren Verständnis sind die bei Herstellung als Zwischenstufen hergestellten Emulsionskonzentrate zusätzlich zu den daraus herstellbaren Tränkemulsionen dargestellt.

Man sieht, dass bereits bei der Herstellung der Emulsionskonzentrate erkannt werden kann, ob letztlich stabile Tränkemulsionen formuliert werden können. Werden keine extrem feinteiligen, mikroemulsionsähnlichen (ME-ähnl.) Emulsionskonzentrate erhalten, so können daraus durch Verdünnung auch keine stabilen Tränkemulsionen hergestellt werden.

Das in den Emulsionen eingesetzte Methyl Glucose Sesquistearate entspricht dem kommerziell erhältlichen Produkt TEGO® Care PS (Degussa).

Das verwendete Polyglyceryl-4 Laurate wurde hergestellt durch Umsetzung von Polyglycerin mit einem mittleren Kondensationsgrad von 4 und Laurinsäure. Der mittlere Veresterungsgrad des Polygylcerins betrug 15 %.

| | Emulsions-konzentrat | 1a Vergleichs- beispiel | 2a Vergleichs-beispiel | 3a | 4a | 5a |
|---|---|---|---|---|---|---|
| A | Polyglyceryl-2 Dipolyhydroxy-stearate | 8,0 % | 7,0 % | | | |
| | Lauryl Glucoside | 8,0 % | 7,0 % | | | |
| | Methyl Glucose Sesquistearate | | | 8,0 % | 7,5 % | 7,0 % |
| | Polyglyceryl-4 Laurate | | | 8,0 % | 7,5 % | 7,0 % |
| | Ethylhexyl Palmitate | 48,0 % | 42,0 % | 48,0 % | 45,0 % | 42,0 % |
| | Phenonip® | 9,0 % | 8,0 % | 9,0 % | 8,4 % | 8,0 % |
| | Parfum | 3,0 % | 2,0 % | 3,0 % | 2,4 % | 2,0 % |
| B | Water | 24,0% | 34,0 % | 24,0 % | 29,2 % | 34,0 % |
| | Aussehen bei 60 bis 70 °C | inhomog. | inhomog. | ME-ähnl. | ME-ähnl. | ME-ähnl. |
| | Verdünnungs-temperatur | 40 °C | 40 °C | 40 °C | 40 °C | 40 °C |

| | Tränkemulsionen | 1 Vergleichs-beispiel | 2 Vergleichs-beispiel | 3 | 4 | 5 |
|---|---|---|---|---|---|---|
| A | Polyglyceryl-2 Dipolyhydroxy-stearate | 0,625 % | 0,625 % | | | |
| | Lauryl Glucoside | 0,625 % | 0,625 % | | | |
| | Methyl Glucose Sesquistearate | | | 0,625 % | 0,625 % | 0,625 % |
| | Polyglyceryl-4 Laurate | | | 0,625 % | 0,625 % | 0,625 % |
| | Ethylhexyl Palmitate | 3,75 % | 3,75 % | 3,75 % | 3,75 % | 3,75 % |
| | Phenonip® | 0,70 % | 0,70 % | 0,70 % | 0,70 % | 0,70 % |
| | Parfum | 0,20 % | 0,20 % | 0,20 % | 0,20 % | 0,20 % |
| | Water (total) | 94,10% | 94,10% | 94,10 % | 94,10 % | |
| | Aussehen | inhomog. | inhomog. | feinteilig | feinteilig | |
| | Stabilität | instabil | instabil | stabil | stabil | |

Die in der unteren Tabelle dargestellten Tränkemulsionen wurden durch Verdünnung der in der oberen Tabelle gekennzeichneten Emulsionskonzentrate hergestellt (1 aus 1a, 2 aus 2a usw.). Wie man erkennen kann, sind die Tränkemulsionen 1 und 2, sowie 3, 4 und 5 jeweils identisch. Allerdings wurden sie über unterschiedliche Emulsionskonzentrate hergestellt.

Man erkennt, dass mit Hilfe der Kombination aus Polyglyceryl-2 Dipolyhydroxystearaten und Lauryl Glucoside keine mikroemulsionsartigen Emulsionskonzentrate erhalten werden konnten, während dies durch Verwendung der erfindungsgemäßen Emulgatorkombinationen problemlos möglich war.

Dementsprechend war es mit dieser Emulgatorkombination auch nicht möglich, langzeitstabile Tränkemulsionen mit der benötigten Menge des Konservierungsmittels Phenonip® herzustellen.

### Emulsionen 6 bis 9:

Anhand der Emulsionen 6 bis 9 wird gezeigt, dass mit Hilfe der erfindungsgemäßen Emulgatorkombination, die auch in den Beispielen 3, 4 und 5 verwendet wurde, auch mit vielen anderen Ölen erfindungsgemäße feinteilige, niedrigviskose Tränkemulsionen hergestellt werden können. Auch in diesem Fall erfolgte die Herstellung durch einfaches Verdünnen der mikroemulsionsähnlichen Emulsionskonzentrate (bei ca. 40 °C mit ca. 25 °C warmen Wasser). Die erhaltenen dünnflüssigen Tränkemulsionen waren ohne weitere Zusätze mehrere Monate lagerstabil.

| | Emulsionskonzentrat | 6a | 7a | 8a | 9a |
|---|---|---|---|---|---|
| A | Methyl Glucose Sesquistearate | 7,5 % | 7,5 % | 7,5 % | 7,5 % |
| | Polyglyceryl-4 Laurate | 7,5 % | 7,5 % | 7,5 % | 7,5 % |
| | Diethylhexyl Carbonate | 45,0 % | | | |
| | Decyl Cocoate | | 45,0 % | | |
| | Cetearyl Ethylhexanoate | | | 45,0 % | |
| | Isopropyl Palmitate | | | | 45,0 % |
| | Phenonip® | 8,4 % | 8,4 % | 8,4 % | 8,4 % |
| | Parfum | 2,4 % | 2,4 % | 2,4 % | 2,4 % |
| B | Water | 29,2 % | 29,2 % | 29,2 % | 29,2 % |
| | Aussehen bei 60 bis 70°C | ME-ähnl. | ME-ähnl. | ME-ähnl. | ME-ähnl. |
| | Verdünnungstemperatur | 40 °C | 40°C | 40 °C | 45 °C |

| | Tränkemulsionen | 6 | 7 | 8 | 9 |
|---|---|---|---|---|---|
| | Methyl Glucose Sesquistearate | 0,625 % | 0,625 % | 0,625 % | 0,625 % |
| | Polyglyceryl-4 Laurate | 0,625 % | 0,625 % | 0,625 % | 0,625 % |
| | Diethylhexyl Carbonate | 3,75 % | | | |
| | Decyl Cocoate | | 3,75 % | | |
| | Cetearyl Ethylhexanoate | | | 3,75 % | |
| | Isopropyl Palmitate | | | | 3,75 % |
| | Phenonip® | 0,7 % | 0,7 % | 0,7 % | 0,7 % |
| | Parfum | 0,2 % | 0,2 % | 0,2 % | 0,2 % |
| | Water (total) | 94,1 % | 94,1 % | 94,1 % | 94,1 % |
| | Aussehen | feinteilig | feinteilig | feinteilig | feinteilig |
| | Stabilität | stabil | stabil | stabil | stabil |

### Emulsionen 10 bis 13:

Die Emulsionen 10 bis 13 zeigen, dass auch andere Polyglycerinpartialester erfindungsgemäß zur Herstellung dünnflüssiger, feinteiliger Öl-in-Wasser Emulsionen benutzt werden können. In den Beispielen wird nur die Zusammensetzung der Tränkemulsionen angegeben. Die Herstellung erfolgte analog zu den Beispielen 1 bis 9 über mikroemulsionsähnliche Emulsionskonzentrate bei Temperaturen von 50 bis 80 °C und anschließendes Verdünnen mit Wasser.

| | Tränkemulsionen | 10 | 11 | 12 | 13 |
|---|---|---|---|---|---|
| | Methyl Glucose Sesquistearate | 0,625 % | 0,625 % | 0,625 % | 0,625 % |
| | Polyglyceryl-4 Laurate* | 0,625 % | | | |
| | Polyglycerl-3 Laurate* | | 0,625 % | 0,625 % | |
| | Polyglyceryl-6 Laurate* | | | | 0,625 % |
| | Diethylhexyl Carbonate | | | 3,75 % | |
| | Isopropyl Palmitate | 3,75 % | 3,75 % | | 3,75 % |
| | Phenonip® | 0,7 % | 0,7 % | 0,7 % | 0,7 % |
| | Parfum | 0,2 % | 0,2 % | 0,2 % | 0,2 % |
| | Water (total) | 94,1 % | 94,1 % | 94,1 % | 94,1 % |
| | Aussehen | feinteilig | feinteilig | feinteilig | feinteilig |
| | Stabilität | stabil | stabil | stabil | stabil |

| | | | | | |
|---|---|---|---|---|---|
| * Die in diesen Beispielen verwendeten Polyglyceryl-3/4/6-Laurate hatten jeweils einen Veresterungsgrad des Polyglycerins von 20 %. | | | | | |

### Emulsionen 14 bis 17:

Anhand der Emulsionen 14 bis 17 wird gezeigt, dass sowohl verschiedene Polyglycerinpartialester als auch unterschiedliche Emulgatoren auf Kohlenhydratbasis zur Herstellung der erfindungsgemäßen Tränkemulsionen eingesetzt werden können.

Die Herstellung erfolgte analog zu den Beispielen 1 bis 13 über mikroemulsionsähnliche Emulsionskonzentrate bei Temperaturen von 50 bis 80 °C und anschließendes Verdünnen mit Wasser.

| | Tränkemulsionen | 14 | 15 | 16 | 17 |
|---|---|---|---|---|---|
| | Methyl Glucose Sesquistearate | 0,375 % | 0,625 % | 0,625 % | 0,875 % |
| | Cetearyl Glucoside | 0,625 % | | | |
| | Polyglyceryl-4 Laurate | 0,125 % | | | |
| | Sorbitan Monooleate | | 0,625 % | 0,583 % | |
| | Polyglyceryl-4 Caprate | | | 0,042 % | 0,375 % |
| | Polyglyceryl-3 Oleate | | | | 0,250 % |
| | Diethylhexyl Carbonate | | | 3,75 % | |
| | Isopropyl Palmitate | | 3,75 % | | |
| | Ethylhexyl Palmitate | 3,75% | | | 3,75 % |
| | Phenonip® | 0,7 % | 0,7 % | 0,7 % | 0,7 % |
| | Parfum | 0,2 % | 0,2 % | 0,2 % | 0,2 % |
| | Water (total) | 94,1 % | 94,1 % | 94,1 % | 93,85 % |
| | Aussehen | feinteilig | feinteilig | feinteilig | feinteilig |
| | Stabilität | stabil | stabil | stabil | stabil |

Bei Cetearyl Glucoside handelt es sich um das kommerziell erhältliche Produkt TEGO® Care CG 90 (Degussa), bei Sorbitan Monooleate um TEGO® SMO V (Degussa), bei Polylglyceryl-4 Caprate um TEGOSOFT® PC 41 (Degussa) und bei Polylglyceryl-3 Oleate um ISOLAN® GO 33 (Degussa).

## Patentansprüche

1. Öl-in-Wasser-Emulsion, enthaltend eine Emulgatorkombination aus Nichtkohlenhydrat-Polyolpartialestern von linearen oder verzweigten Fettsäuren mit 6 bis 22 C-Atomen (Emulgatorkomponente A) und Emulgatoren auf Kohlenhydratbasis (Emulgatorkomponente B), ein oder mehrere Öle, mindestens 10 Gew.-% Konservierungsmittel bezogen auf die Gesamtmenge an Emulgatorkomponenten A und B.

2. PEG-freie Öl-in-Wasser-Emulsion, enthaltend eine Emulgatorkombination aus Nichtkohlenhydrat-Polyolpartialestern von linearen oder verzweigten, gesättigten oder ungesättigten Fettsäuren mit 6 bis 22 C-Atomen (Emulgatorkomponente A) und Emulgatoren auf Kohlenhydratbasis (Emulgatorkomponente B), ein oder mehrere Öle sowie Konservierungsmittel.

3. Öl-in-Wasser-Emulsion nach Patentanspruch 1 und/oder 2, **dadurch gekennzeichnet, dass** sie 20 bis 75 Gew.-% Konservierungsmittel, bezogen auf die Gesamtmenge an Emulgatorkomponenten A und B enthält.

4. Öl-in-Wasser-Emulsion nach einem oder mehreren der Patentansprüche 1 bis 3, **dadurch gekennzeichnet, dass** sie zusätzlich Co-Emulgatoren und übliche Hilfs- und Zusatzstoffe enthält.

5. Öl-in-Wasser-Emulsion nach einem oder mehreren der Patentansprüche 1 bis 4, **dadurch gekennzeichnet, dass** Emulgatorkomponente A ausschließlich oder teilweise Polyglycerinpartialester umfasst, die durch Umsetzung von Polyglycerinen mit linearen oder verzweigten Fettsäuren mit 6 bis 22 C-Atomen erhältlich sind.

6. Öl-in-Wasser-Emulsion nach Patentanspruch 5, **dadurch gekennzeichnet, dass** das eingesetzte Polyglyceringemisch einen mittleren Kondensationsgrad von 2 bis 10, insbesondere 2 bis 8 und speziell 3 bis 6 aufweist.

7. Öl-in-Wasser-Emulsion nach Patentanspruch 5 oder 6, **dadurch gekennzeichnet, dass** der Veresterungsgrad des eingesetzten Polyglyceringemischs dabei zwischen 5 und 70 %, insbesondere zwischen 10 und 30 % liegt, bezogen auf die ursprünglichen Hydroxylgruppen des Polyols.

8. Öl-in-Wasser-Emulsion nach einem oder mehreren der Patentansprüche 1 bis 7, **dadurch gekennzeichnet, dass** die Emulgatorkomponente A teilweise oder vollständig durch Sorbitanmono- und/oder -diester von ungesättigten und gesättigten Fettsäuren mit 6 bis 22 C-Atomen gebildet wird.

9. Öl-in-Wasser-Emulsion nach Patentanspruch 8, **dadurch gekennzeichnet, dass** Emulgatorkomponente A neben einem oder mehreren Polyglycerinpartialestern bis zu 50 Gew.-% Sorbitanester enthält, bezogen auf die Gesamtmenge an Emulgatorkomponente A.

10. Öl-in-Wasser-Emulsion nach einem oder mehreren der Patentansprüche 1 bis 9, **dadurch gekennzeichnet, dass** eine oder mehrere verschiedene Emulgatorkomponenten B aus den folgenden Gruppen ausgewählt ist/sind
i) Ester aus Mono- und/oder Polysacchariden und einer oder mehreren linearen oder verzweigten Fettsäuren mit 6 bis 22 C-Atomen,
ii) Glykoside aus Mono- oder Polysacchariden und linearen oder verzweigten Fettalkoholen mit 6 bis 22 C-Atomen,
iii) Glykoside, die zusätzlich mit linearen oder verzweigten Fettalkoholen mit 1 bis 22 C-Atomen, verethert worden sind,
iv) Glykosidester aus Mono- oder Polysacchariden und linearen oder verzweigten Fettalkoholen mit 1 bis 22 C-Atomen und einer oder mehreren linearen oder verzweigten Fettsäuren mit 1 bis 22 C-Atomen.

11. Öl-in-Wasser-Emulsion nach einem oder mehreren der Patentansprüche 1 bis 10, **dadurch gekennzeichnet, dass** die Alkylpolyglykoside im Schnitt 1 bis 5 Zuckereinheiten, insbesondere 1,1 bis 2 Zuckereinheiten enthalten.

12. Öl-in-Wasser-Emulsion nach einem oder mehreren der Patentansprüche 1 bis 11, **dadurch gekennzeichnet, dass** die Emulgatorkomponenten A und B in einem Gewichtsverhältnis von 9:1 bis 1:9, insbesondere von 8:2 bis 2:8 und ganz besonders von 7:3 bis 3:7 eingesetzt werden.

13. Öl-in-Wasser-Emulsion nach einem oder mehreren der Patentansprüche 1 bis 12, **dadurch gekennzeichnet, dass** die Gesamtmenge der eingesetzten Emulgatorkomponenten A + B bezogen auf die zu emulgierende Ölmenge in einer Spanne von 15 bis 70 Gew.-%, bevorzugt von 25 bis 55 Gew.-% und besonders bevorzugt von 30 bis 50 Gew.-% liegt.

14. Öl-in-Wasser-Emulsion nach einem oder mehreren der Patentansprüche 1 bis 13, **dadurch gekennzeichnet, dass** die Gesamtmenge an Ölphase (Emulgator + Öl) im Bereich von 1 bis 25 Gew.-%, insbesondere im Bereich von 1,5 bis 10 Gew.-% Ölphase und ganz besonders im Bereich von 2 bis 6 Gew.-% liegt.

15. Öl-in-Wasser-Emulsion nach einem oder mehreren der Patentansprüche 1 bis 14, **dadurch gekennzeichnet, dass** die enthaltenen Konservierungsmittel Mischungen von Alkylparabenestern mit Phenoxyethanol umfassen.

16. Verfahren zur Herstellung feinteiliger, niedrigviskoser Öl-in-Wasser Emulsionen gemäß einem oder mehreren der Patentansprüche 1 bis 15, wobei man zunächst bei erhöhten Temperaturen ein Emulsionskonzentrat herstellt und anschließend mit Wasser verdünnt.

17. Verfahren gemäß Patentanspruch 16, **dadurch gekennzeichnet, dass** man zunächst ein 30 bis 90 %iges transparentes Emulsionskonzentrat bei Temperaturen zwischen 40 und 85 °C herstellt.

18. Verfahren gemäß Patentanspruch 16 oder 17, **dadurch gekennzeichnet, dass** man die Emulsionskonzentrate in eine vorgelegte Wasserphase von 20 bis 50 °C einrührt.

19. Im Bereich von 50 bis 65 °C im Wesentlichen transparentes Konzentrat, enthaltend 30 bis 90 Gew.-%, besonders bevorzugt 50 bis 80 Gew.-% einer Ölphase, bestehend im Wesentlichen aus Ölkomponente, Emulgatorkomponenten und Konservierungsmittel und ad 100 Gew.-% einer Wasserphase, wobei das Konzentrat im Wesentlichen PEG-frei ist.

20. Im Bereich von 50 bis 65 °C im Wesentlichen PEG-freies, transparentes Konzentrat, enthaltend 30 bis 90 Gew.-% einer PEG-freien Emulgatorkombination aus Nichtkohlenhydrat-Polyolpartialestern von linearen oder verzweigten Fettsäuren mit 6 bis 22 C-Atomen (Emulgatorkomponente A) und Emulgatoren auf Kohlenhydratbasis (Emulgatorkomponente B), ein oder mehrere Öle sowie Konservierungsmittel.

21. Konzentrat nach Patentanspruch 19 oder 20, **dadurch gekennzeichnet, dass** es 15 bis 70 Gew.-% der Emulgatorkombination enthält.

22. Verwendung einer Öl-in-Wasser-Emulsion gemäß einem der Ansprüche 1 bis 15 zur Herstellung von kosmetischen, dermatologischen oder pharmazeutischen Zubereitungen, insbesondere zur Herstellung von Feuchttüchern.

23. Verwendung gemäß Patentanspruch 22 zur Herstellung von kosmetischen Feuchttüchern zur Pflege und Reinigung.

24. Verwendung gemäß Patentanspruch 22 zur Herstellung von kosmetischen Reinigungs- und Pflegezubereitungen für Haut- und Hautanhangsgebilde.

25. Verwendung gemäß Patentanspruch 22 zur Herstellung von Sonnenschutzformulierungen und von selbstbräunenden Zubereitungen.

26. Verwendung gemäß Patentanspruch 22 zur Herstellung von Reinigungs- und Pflegemitteln für harte Oberflächen.
